(19) Europäisches Patentamt
European Patent Office
Office européen des brevets

(11) Veröffentlichungsnummer: **0 168 659**
**B1**

(12) # EUROPÄISCHE PATENTSCHRIFT

(45) Veröffentlichungstag der Patentschrift: **07.12.88**

(51) Int. Cl.⁴: **C 07 D 251/32**, C 07 D 251/36

(21) Anmeldenummer: **85107539.0**

(22) Anmeldetag: **19.06.85**

(54) **Verfahren zur Herstellung von Chlorisocyanursäuren.**

(30) Priorität: **06.07.84 DE 3424823**

(43) Veröffentlichungstag der Anmeldung: **22.01.86 Patentblatt 86/4**

(45) Bekanntmachung des Hinweises auf die Patenterteilung: **07.12.88 Patentblatt 88/49**

(84) Benannte Vertragsstaaten: **BE CH DE FR GB IT LI NL**

(56) Entgegenhaltungen:
**DE-C- 1 072 625**
**DE-C- 1 141 641**
**GB-A- 1 388 936**
**US-A- 2 969 360**

**FETTE, SEIFEN, ANSTRICHMITTEL, Band 63, Nr. 5, 1961, Seiten 451-455, Leinfelden-Echterdingen; K. LINDNER "Die Chlorisocyanursäuren und ihre Salze als Bestandteile neuzeitlicher Wasch-, Bleich- und Desinfektionsmittel"**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Pieper, Werner, Dr., Am Käferbruch 14, D-5014 Kerpen (DE)**

**EP 0 168 659 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Chlorisocyanursäuren durch Umsetzung von einer wässrigen Cyanursäuresuspension unter Rühren mit einer Alkaliverbindung in Gegenwart von Chlorgas bei Temperaturen zwischen 0 und 40°C, vorzugsweise zwischen 10 und 20°C, und pH-Werten unter 7.

Die Herstellung der Chlorisocyanursäuren erfolgt bekanntermassen durch Umsetzung wässriger Lösungen bzw. Suspensionen von Cyanursäuresalzen, bzw. Cyanursäure-Base-Gemischen mit gasförmigem oder flüssigem Chlor, wobei der Chlorierungsgrad durch die Wahl des Stoffmengenverhältnisses Cyanursäure zu Base festgelegt wird (Fette, Seifen, Anstrichmittel 63, 451 [1961]).

$$C_3H_3N_3O_3 + n\,MOH + n\,Cl_2 \rightarrow C_3H_{3-n}Cl_nN_3O_3 + n\,MCl + n\,H_2O$$

M = Alkalimetall, n = 1 bis 3

Als Produkt resultiert eine Suspension der Chlorisocyanursäure in einer wässrigen Chloridlösung.

Gemäss der US-PS 29 69 360 gelten als günstigste Bedingungen für die Chloraufnahme durch die Reaktionsmischung: niedrige Temperaturen, hoher pH-Wert, effektives Verteilen des Chlorgases in der Reaktionsmischung und gutes Durchmischen der Reaktionslösung.

In alkalischer Lösung erfolgt jedoch ein oxidativer Abbau der Cyanursäure gemäss folgender Reaktionsgleichung, so dass diese Reaktionsführung prinzipiell gegenüber der Chlorierung in saurer Phase benachteiligt ist:

$$2\,C_3H_3N_3O_3 + 9\,Cl_2 + 18\,NaOH \rightarrow 6\,CO_2 + 3\,N_2 + 18\,NaCl + 12\,H_2O$$

In saurer Lösung dagegen ist die Chlorabsorption im Reaktionsgemisch nicht quantitativ, so dass ein Teil des eingeleiteten Chlorgases ungenutzt bleibt und als Abgas anfällt.

Bevorzugt wird daher ein Zweistufenprozess, bei dem das Abgas der sauren zweiten Stufe in eine alkalische erste Chlorierungsstufe eingeleitet wird. In der ersten Stufe werden dabei die eingesetzten Edukte (Cyanursäure und Alkalihydroxid) partiell chloriert und in die zweite Stufe zur vollständigen Chlorierung überführt.

Die DE-PS 1 072 625 beschreibt ein Herstellverfahren für chlorierte Cyanursäuren, dem zufolge während der gesamten Reaktion der pH-Wert einer stark verdünnten Cyanursäuresuspension unter 7 gehalten wird. Statt eines Alkalihydroxids werden jedoch Natriumsalze wie Natriumphosphat oder Natriumacetat in dem für den Chlorierungsgrad notwendigen stöchiometrischen Verhältnis eingesetzt und der Anfangs-pH-Wert durch Zugabe von Säure oder Base auf einen, den Chlorierungsgrad bestimmenden Bereich eingestellt. Damit fällt aber nach dem Abfiltrieren der chlorierten Cyanursäure neben den unvermeidlichen, stöchiometrischen Chloridmengen eine Mutterlauge an, die zusätzlich mit den eingesetzten Mengen von Salzen wie Phosphaten, Acetaten usw. belastet ist.

Ferner erfolgt beim Einleiten von Chlorgas in eine alkalische Suspension von Cyanursäure mit der für den gewünschten Chlorierungsgrad erforderlichen Menge Natriumhydroxid im Bereich des Neutralpunktes, d.h. wenn die restliche Stoffmenge der Base der Stoffmenge an Cyanursäure entspricht, eine starke Entwicklung von Kohlendioxid und Stickstoff. Die freigesetzten Reaktionsgase zusammen mit nicht umgesetztem Chlorgas flotieren die aus der Reaktionsmischung sich abscheidenden Feststoffteilchen an die Oberfläche und bilden einen Schaum, der nur schwer in die Suspension zurückgebracht werden kann und die Reaktorausgänge blockiert. Die Folge ist eine verminderte Ausbeute bzw. ein zu geringer Chlorierungsgrad des Endprodukts.

Überraschenderweise wurde nun gefunden, dass sich die oben genannten Schwierigkeiten in einfacher Weise überwinden lassen, wenn man

a) die Umsetzung der wässrigen Cyanursäuresuspension in einem nach aussen gasdicht abgeschlossenen System durchführt;

b) über der sich durch Rühren ständig erneuernden Oberfläche der Cyanursäuresuspension eine Chlorgasatmosphäre mit einem Druck grösser als 500 mbar – vorzugsweise mit einem Überdruck von 50 bis 500 mbar, bezogen auf die Aussenatmosphäre – einstellt und während der Umsetzung aufrecht erhält und

c) als Alkaliverbindung eine Alkalihydroxidlösung einsetzt, die man mit einer solchen Geschwindigkeit zu der Cyanursäure dosiert, dass der pH-Wert des Reaktionsgemisches ständig unter 7 gehalten wird.

Im Gegensatz zu allen bisher üblichen Arbeitsweisen wird dabei das Chlor nicht direkt in die Cyanursäuresuspension sondern nur in den Gasraum des Reaktors eingeleitet, so dass es lediglich an der Oberfläche der Suspension absorbiert und umgesetzt wird.

Als Alkalimetallhydroxidlösung wird bevorzugt Natronlauge eingesetzt. Die Reaktionstemperatur wird durch Kühlen des Reaktors zwischen 0 und 40°C, vorzugsweise zwischen 10 und 20°C, gehalten.

Die Vorteile des erfindungsgemässen Verfahrens gegenüber den bekannten Arbeitsweisen sind:

Es entstehen keine gasförmigen Nebenprodukte, insbesondere Kohlendioxid und Stickstoff, da der pH-Wert der Reaktionsmischung stets im sauren Bereich bleibt und damit die oben genannte Zersetzungsreaktion nicht ablaufen kann.

Eine Überdosierung der Natronlauge, wie sie in der US-PS 29 69 360 empfohlen wird, um eine vollständige Chlorierung zu erreichen, ist nicht erforderlich, da kein Alkali für Nebenreaktionen verbraucht wird.

Da das Chlorgas nicht in die Suspension eingeleitet, sondern nur an der Oberfläche der Mischung absorbiert wird und da ferner kein $CO_2$ und $N_2$ als Zersetzungsprodukte auftreten, unterbleibt die störende Schaumbildung vollständig. Dennoch ist die Reaktionsgeschwindigkeit hoch, da durch Rühren der Suspension die Oberfläche ständig erneuert wird.

Das einstufige Verfahren bedarf eines geringen apparativen Aufwands. Der Reaktor kann verhältnismässig klein dimensioniert werden, da kein Sicherheitsvolumen wegen Schaumbildung benötigt wird.

Obwohl die Reaktion im sauren Reaktionsmedium durchgeführt wird, ist die Chlorausbeute quantitativ, da durch Absperren des Reaktionssystems gegen die Umgebung kein Chlorgas verlohren geht.

Als Absperreinrichtung hierzu sind z.B. ein Druckventil oder eine Tauchung geeignet, so dass die Chlorierung bei einem konstanten Druck, vorzugsweise einem geringen Überdruck, durchgeführt werden kann.

Die Chlorabsorption ist bei konstanter Rührgeschwindigkeit und konstantem Druck proportional der Alkalihydroxiddosierung. Das Stoffmengenverhältnis zwischen absorbiertem Chlor und zudosiertem Alkalihydroxid beträgt während der gesamten Reaktionszeit 0,9 bis 1,1.

Die Ausbeute an Chlorisocyanursäuren ist praktisch quantitativ und wird lediglich durch die Löslichkeit des Endprodukts in der Mutterlauge begrenzt.

Die Konzentration der Reaktionskomponenten ist so zu wählen, dass während der gesamten Reaktion ein gut rührbares Reaktionsgut vorhanden ist. Das ist gewährleistet, wenn eine ca. 10 bis 20%ige Cyanursäuresuspension eingesetzt, bzw. eine 10 bis 20%ige Chlorisocyanursäuresuspension als Produkt erhalten wird.

Im Falle der Dichlorisocyanursäure kann durch Neutralisation mit einer wässrigen Base wie Natronlauge das entsprechende Salz in bekannter Weise hergestellt werden.

Die Vorteile der erfindungsgemässen Arbeitsweise gegenüber den bisher bekannten Herstellungsverfahren seien anhand der folgenden Beispiele verdeutlicht.

Beispiel 1

In einem 10-l-Mehrhalskolben mit Rührer, Tropftrichter, Innenthermometer und Anschluss an eine Chlorgasflasche wird 1 kg (7,75 mol) Cyanursäure in Form einer 16,7 gew%igen wässrigen Suspension in einer Chloratmosphäre von 100 mbar Überdruck vorgelegt. Das Reaktionssystem ist gegen die Umgebung durch eine wassergefüllte Tauchung abgetrennt, die einen maximalen Überdruck von 200 mbar zulässt. In die gerührte Suspension werden unter Kühlung auf 10 bis 20°C 1550 g 40%ige Natronlauge mit einer Geschwindigkeit von 17 g pro Minute eindosiert, wobei die Chlorgaszuführung so erfolgt, dass der Druck im Reaktionssystem konstant bleibt. In 100 min werden 1100 g (15,5 mol) Chlor verbraucht, danach nimmt die Suspension kein weiteres Chlorgas auf. Das Produkt wird abfiltriert, mit Wasser gewaschen und getrocknet. Die Ausbeute beträgt 1421 g (7,2 mol) Dichlorisocyanursäure, entsprechend 92,9% der theoretischen Menge, mit einem Aktivchlorgehalt von 70,5%.

Beispiel 2

In einer Apparatur analog Beispiel 1 wird eine Suspension von 645,5 g (5 mol) Cyanursäure in 3230 g Wasser unter Kühlung auf 10 bis 20°C in einer Chloratmosphäre mit 100 mbar Überdruck gerührt. 1500 g 40 gew%ige Natronlauge werden gleichmässig mit einer Geschwindigkeit von 10,8 g pro Minute zudosiert. Die Chloraufnahme beträgt 3,4 g pro Minute, so dass nach ca. 150 min bei einer Gesamtchloraufnahme von 1100 g (15,5 mol) $Cl_2$ die Reaktion beendet ist. Das Stoffmengenverhältnis von absorbiertem Chlor und zudosierter Natronlauge beträgt während der gesamten Reaktion 0,9 bis 1,1. 1090 g (4,7 mol) Trichlorisocyanursäure werden durch Filtration abgetrennt, was einer Ausbeute von 94% entspricht.

Beispiel 3 (Vergleichsbeispiel)

In einer Apparatur analog Beispiel 1 werden 1024 g (7,94 mol) Cyanursäure mit 6040 g Wasser und 667 g (16,7 mol) Natriumhydroxid (5,2 Gew% Überschuss) in einer Chloratmosphäre von 50 mbar vorgelegt. Nach dem Einschalten des Rührers werden innerhalb von 45 min 0,8 mol Chlor pro Mol Cyanursäure aufgenommen. Danach verlangsamt sich die Chloraufnahme drastisch. Die Entfärbung des Gasraums über der Reaktionsmischung zeigt an, dass ein farbloses Gas entsteht, welches die Chloraufnahme behindert. Nur durch mehrmaliges Ablassen des Fremdgases kann die Chlorierung weitergeführt werden, bis bei einem Umsetzungsgrad von ca. 1,5 mol Chlor pro Mol Cyanursäure die Fremdgasentwicklung endet. Insgesamt 20 l Abgas wurden aufgefangen, das laut Gaschromatogramm aus Kohlendioxid und Stickstoff besteht. Im weiteren Verlauf der Reaktion wird kein Fremdgas mehr gebildet. Der Gesamtverbrauch an Chlor beträgt 1200 g (16,9 mol). 1200 g (6,06 mol) Dichlorisocyanursäure werden isoliert, was einer Ausbeute von 76,3% entspricht.

**Patentansprüche**

1. Verfahren zur Herstellung von Chlorisocyanursäuren durch Umsetzung einer wässrigen Suspension von Cyanursäure unter Rühren mit einer Alkaliverbindung in Gegenwart von Chlorgas bei Temperaturen zwischen 0 und 40°C und pH-Werten unter 7, dadurch gekennzeichnet, dass man

a) die Umsetzung in einem nach aussen gasdicht abgeschlossenen System durchführt;

b) über die sich durch Rühren ständig erneuernden Oberfläche der Cyanursäuresuspension eine Chlorgasatmosphäre mit einem Druck grösser als 500 mbar einstellt und während der Umsetzung aufrechterhält und

c) als Alkaliverbindung eine Alkalihydroxidlösung einsetzt, die man mit einer solchen Geschwindigkeit zu der Cyanursäuresuspension dosiert, dass der pH-Wert des entstehenden Reaktionsgemisches ständig unter 7 gehalten wird.

2. Verfahren nach Anspruch 1, dadurch gekennzeichnet, dass man in der Chlorgasatmosphäre einen Überdruck von 50 bis 500 mbar, bezogen auf die Aussenatmosphäre, einstellt und aufrechterhält.

3. Verfahren nach Anspruch 1 oder 2, dadurch gekennzeichnet, dass man als Ausgangsprodukt eine 10 bis 20%ige Cyanursäuresuspension einsetzt.

**Claims**

1. A process for the preparation of chloroisocyanuric acids by reaction of an aqueous suspension of cyazbruc acid with an alkali metal compound with stirring in the presence of chlorine gas at temperatures of between 0 and 40°C and pH values below 7, which comprises
a) carrying out the reaction in a system which is sealed gas-tight toward the outside;
b) establishing and maintaining during the reaction a chlorine-gas atmosphere having a pressure greater than 500 mbar by means of the surface of the cyanuric acid suspension which is constantly renewed by stirring and
c) using as the alkali metal compound an alkali metal hydroxide solution which is metered into the cyanuric acid suspension at such a rate that the pH of the resulting reaction mixture is constantly maintained below 7.

2. The process as claimed in claim 1, where in a superatmospheric pressure of 50 to 500 mbar, relative to the outside atmosphere, is established and maintained in the chlorine gas atmosphere.

3. The process as claimed in claim 1 or 2, wherein in the starting product used is a 10 to 20% strength cyanuric acid suspension.

**Revendications**

1. Procédé de préparation d'acides chlorisocyanuriques par réaction d'une suspension aqueuse d'acide cyanurique sous agitation avec un composé alcalin, en présence de chlore gazeux, à des températures de 0 à 40°C et des pH inférieurs à 7, caractérisé en ce que:
a) on effectue la réaction dans un système clos, étanche aux gaz par rapport à l'extérieur;
b) on règle et on maintient pendant toute la réaction une atmosphère de chlore gazeux à une pression supérieure à 500 mbar sur la surface en renouvellement permanent sous l'agitation de la suspension d'acide cyanurique, et
c) on utilise en tant que composé alcalin une solution d'hydroxyde alcalin qu'on introduit dans la suspension d'acide cyanurique à une vitesse telle que le pH du mélange de réaction reste en permanence au-dessous de 7.

2. Procédé selon la revendication 1, caractérisé en ce que l'on règle et on maintient dans l'atmosphère de chlore gazeux une surpression den 50 à 500 mbar par rapport à l'atmosphère extérieure.

3. Procédé selon la revendication 1 ou 2, caractérisé en ce que le produit de départ utilisé est une suspension d'acide cyanurique à une concentration de 10 à 20%.